# EUROPÄISCHE PATENTSCHRIFT

(11) **EP 1 678 479 B1**
(45) Veröffentlichungstag und Bekanntmachung des Hinweises auf die Patenterteilung: **05.07.2017**
(21) Anmeldenummer: 04761077.9
(22) Anmeldetag: 21.10.2004
(51) Int. Cl.: G01N 3/08, G01N 33/46, G01L 1/22, G01L 1/24, G01B 7/16, G01B 11/16

(54) **VERFAHREN ZUR QUALITÄTSSICHERUNG VON LANGHOLZ**
METHOD FOR QUALITY ASSURANCE OF TIMBER
PROCEDE D'ASSURANCE DE LA QUALITE DE BOIS DE CONSTRUCTION

(30) Priorität: 27.10.2003 AT 16992003; 22.06.2004 AT 10552004; 22.06.2004 AT 10542004
(43) Veröffentlichungstag der Anmeldung: 12.07.2006
(73) Patentinhaber: Leitinger, Hans-Peter, 8551 Wies (AT)
(72) Erfinder: LEITINGER, Hans-Peter, 8551 Wies (AT); LEITINGER, Sebastian, 8551 Wies (AT)
(74) Vertreter: Schwarz & Partner Patentanwälte OG
(86) Internationale Anmeldenummer: PCT/AT2004/000362
(87) Internationale Veröffentlichungsnummer: WO 2005/040766

(56) Entgegenhaltungen:
- DE-A1- 3 412 675
- DE-A1- 4 012 444
- DE-A1- 4 140 349
- US-A- 5 237 870

## Beschreibung

Die Erfindung betrifft ein Verfahren zur Qualitätssicherung von in Serie erzeugtem, vorzugsweise keilverzinktem, Langholz aus Kantholz, vorzugsweise aus Konstruktionsvollholz, mit vorbestimmter Mindestlänge.

Um aus Baumstämmen qualitativ hochwertiges Langholz, wie es z.B. als Bauholz Verwendung findet, zu fertigen und hierbei eine einigermaßen gleichmäßige Qualität sicherzustellen, werden die Baumstämme auf das gewünschte Maß geschnitten bzw. formatiert und, falls die so zugeschnittenen Rohlinge Fehlstellen, alias Schwachstellen, wie Durchfalläste etc. enthalten, werden diese Fehlstellen bzw. Schwachstellen entfernt und die verbleibenden Rohlingstücke mittels stirnseitiger Keilzinkenstöße zu Langholz verklebt. Auf diese Art und Weise wird Brettschichtholz, das aus mehreren Lagen von längsverzinkten, miteinander verklebten und versetzt Keilzinkenstöße aufweisenden Brettern gebildet ist, gefertigt. Balkenschichtholz wird aus zwei bis drei miteinander längsverklebten Balken, die gegebenenfalls ebenfalls aus Teilstücken zusammengesetzt sind, die mittels Keilzinkenverbindungen verbunden sind, gefertigt.

Ein besonderes Problem bildet die Verarbeitung von Starkholz, worunter Bäume verstanden werden, die in Brusthöhe über ca. 50 cm Durchmesser aufweisen. Solches Starkholz weist den Vorteil einer höheren Ausbeute auf, und zwar dann, wenn es zu Konstruktionsvollholz verarbeitet wird. Allerdings sind die Holzeigenschaften sehr heterogen, d.h. das Starkholz bedingt einen höheren Selektionsaufwand. Zudem kann ein Nasskern oder ein Kernriss Probleme verursachen. Starke Äste bewirken ebenfalls schlechtere mechanische Eigenschaften. Aus diesem Grund wird aus Starkholz gefertigtes Langholz nur selten einteilig aus einem Stamm geschnitten werden können; meist ist es notwendig, Schwachstellen herauszuschneiden und die Teilstücke, wie oben erwähnt, mittels einer Keilzinkenverbindung zu einem Langholz zu verkleben.

Es ist bekannt, diesen Prozess mehr oder weniger automatisiert durchzuführen, wobei das Holz zunächst eine Qualitätssortieranlage durchläuft, in der es auf Feuchtigkeit, Jahresringdichte, Farb- und Strukturbeschaffenheit, Äste, etc. untersucht wird, was entweder visuell erfolgen kann oder durch elektrische Widerstandsmessung (für die Feuchtigkeitsmessung) oder mittels Laserkameras. Zum Feststellen versteckter Äste wird die Röntgen- oder Computertomographie- oder Ultraschalltechnologie herangezogen. Das Herausschneiden der Fehlstellen sowie das Verkleben der Keilzinkenverbindung erfolgen meist in automatisch gesteuerten Anlagen.

Zur Sicherung der Güte von in solchen Anlagen gefertigten Keilzinkenverbindungen werden Zerstörungsproben durchgeführt, wobei ein Bruch bei einer für eine solche Zerstörungsprobe vorgesehenen Biegeprobe nicht im Bereich der Keilzinkenverbindung auftreten darf.

Es hat sich gezeigt, dass trotz der automatisierten Fehlererkennungsmethoden und trotz nachfolgender eingehender visueller Besichtigung bei der Verwendung von solcherart hergestelltem Langholz unerwartet Brüche - z.B. verursacht durch Stauchbrüche z.B. bei Windbruch, Verleimungsfehler, Verzahnungsfehler, innere Risse etc. - auftreten können, sodass es Bestrebungen gibt, Konstruktionsvollholz von Holzkonstruktionen, bei denen dem Holz eine tragende Funktion zukommt, auszuschließen. Dies ist insbesondere von Nachteil, als hierdurch eine kostengünstige Verwertung als Kantholz aus Starkholz nicht mehr gegeben wäre, d.h. das Starkholz müsste ebenfalls zu mehrlagigem Brettschichtholz oder Balkenschichtholz verarbeitet werden, bei denen verdeckte bzw. nicht aufgefundene Fehlstellen bzw. Schwachstellen in einzelnen Teilstücken in Folge der mehrlagigen Verleimung weniger ins Gewicht fallen.

Die Erfindung stellt sich die Aufgabe, ein Verfahren zur Qualitätssicherung von in Serie erzeugten Langhölzern oder Bohlen zu schaffen, um diese auch für höherbeanspruchte Konstruktionsteile effizient einsetzen zu können. Insbesondere sollen die derzeit aufgrund der Inhomogenität des Holzes erforderlichen Querschnittszuschläge stark herabgesetzt werden können.

Da in den Wäldern Starkholz überwiegt und der Anteil an Starkholz noch im Steigen begriffen ist, stellt sich die Erfindung die spezielle Aufgabe, ein Verfahren zur Qualitätssicherung zu finden, um gerade dieses Starkholz ebenfalls mit der erforderlichen Sicherheit für hochbelastete Konstruktionen einsetzen zu können, in denen Langholz tragende Funktionen übernimmt, wobei das Langholz als Konstruktionsvollholz ausgebildet ist, d.h. nicht aus einzelnen miteinander längsverleimten Holzschichten gebildet ist.

Diese Aufgabe wird erfindungsgemäß dadurch gelöst, dass jedes in der Länge fertig hergestellte Langholz jeweils mit an seinen Enden angreifenden und bis zu einem Grenzwert unterhalb der Bruchlast eines fehlerfreien Langholzes ansteigenden Zugkräften belastet wird.

Die Erfindung beruht auf der Erkenntnis, dass bei einer Zugprüfung von Holz unterhalb der Bruchgrenze aufgrund der strengen Linearität zwischen Längenänderung und Spannung keine Verschlechterung der Holzeigenschaften auftritt.

Vorzugsweise wird eine bei ansteigender Belastung mit Zugkräften ansteigende Längenänderung in mindestens einem Abschnitt oder über die gesamte Länge des Langholzes bestimmt und als Qualitätskriterium für die Verwendung des Konstruktionsvollholzes bzw. dessen Weiterbehandlung herangezogen

Hierbei werden vorzugsweise zur Bestimmung der Längenänderung das Laser-Speckle Verfahren oder ein Differenzlängenmessverfahren mit direkter Messmethode oder indirekten Messmethoden, wie z.B. eine Widerstandsänderung von Dehnmessstreifen, angewendet.

Kommt es bei der Zugprüfung zu einem Bruch des Langholzes, wird vorzugsweise die beim Bruch ermittelte Zugspannung für eine Güteklassifizierung der Bruchstücke für deren weitere Verwendung oder Weiterbehandlung herangezogen.

Eine bevorzugte Variante ist dadurch gekennzeichnet, dass bei Feststellen einer unzulässig hohen Längenänderung des Langholzes bei vorgegebener Zugkraft das Langholz aus der Produktion ausgeschleust, die die unzulässige Längenänderung verursachende Schwachstelle des Langholzes bzw. die Schwachstellen des Langholzes herausgeschnitten und die verbleibenden schwachstellenfreien Teile des Langholzes mittels Keilverzinkung zu einem neuen Langholz, gegebenenfalls unter Hinzufügung weiterer Langholzteile, zusammengefügt werden, worauf das neu hergestellte Langholz abermals dem Verfahren nach Anspruch 1 unterworfen wird.

Unter Ausnutzung der oben angegebenen Erkenntnis kann im Produktionsablauf ein Zugversuch bis zur Bruchgrenze durchgeführt (Bruchgrenzen-Feststellprüfung) werden. Das Prüfprotokoll zeigt die Bruchspannung. Bei Beseitigung der Bruchstelle durch Kappen nach dem Bruch und mögliche Wiederverbindung durch z.B. eine Keilverzinkung der Bruchstücke kann (bei ordnungsgemäßer Verzinkung) bei einer Wiederholung des Zugversuches bis maximal zur Bruchgrenze kein Wiederholungsbruch auftreten, da alle anderen Stellen des Holzes die Prüfung bereits schadlos überstanden haben. Um die volle Materiallänge zu bewahren, kann nach Beseitigung des Bruchabschnittes ein hochqualitativer Neuteil eingefügt werden. Dabei entsteht jedoch ein größeres Risiko für einen Wiederholungsbruch (zwei Verzinkungsstellen, ungetestetes Neumaterial). Zweckmäßig wird der zweite Zugversuch mit einer stark verminderten Zuglast, aber für die gewünschte Festigkeitserfordernis ausreichender Belastung, durchgeführt. Bei Bruchstücken mit ausreichender Größe für die Weiterbearbeitung ist nach Kappung der Bruchenden das so erhaltene Material direkt als fertig geprüft ansehbar, und eine zweite Zugprüfung kann entfallen.

Durch diese Maßnahmen gelingt ein Wieder-Einbinden von bei der Zugprüfung ausgeschiedenem Langholz in den Produktionsprozess, so dass eine optimale Holzverwertung bei geringstem Abfall bzw. Verschnitt gegeben ist.

Zweckmäßig werden die Zugkräfte auf das Langholz über an den Enden des Langholzes an zwei einander gegenüberliegenden Seiten vorgesehenen Klemmbacken auf das Langholz aufgebracht.

Vorteilhaft wird jedes geprüfte Langholz mit einer Qualitätsbezeichnung versehen.

Die Fertigbearbeitung des Langholzes, wie z.B. durch Hobeln, Schleifen oder Fräsen, wird bevorzugt nach der Prüfung durchgeführt.

Es hat sich von Vorteil erwiesen, dass in Abhängigkeit des Ergebnisses der Längenänderungsbestimmung die Langhölzer mindestens zwei unterschiedlichen Qualitätsklassen zugeordnet werden, wodurch es möglich ist, für jede spezielle Anforderung hinsichtlich Festigkeitswerte das günstigste Produkt bereitzustellen und auch nicht den höchsten Ansprüchen genügendes Langholz effizient zu verwenden.

Vorzugsweise wird das Rohmaterial nach einer Oberflächenanalyse ungeteilt und/oder in Teilstücken geteilt in Güteklassen sortiert und in jeder Güteklasse für sich als Langholz, gegebenenfalls nach Keilverzinken, einem Verfahren zur Qualitätsricherung nach Anspruch 1 unterzogen.

Es ist eine weitere Aufgabe der Erfindung, ein Verfahren zu schaffen, mit dem für Holzkonstruktionen aus qualitätsgesichertem Langholz Balken bzw. Binder herstellbar sind, die sehr große Querschnitte aufweisen und hierbei voll eine tragende Funktion übernehmen können, und zwar mindestens gleichwertig einem Leimbinder aus Brettschichtholz, und vorzugsweise sogar höher belastbar sind als diese.

Diese spezielle Aufgabe wird erfindungsgemäß dadurch gelöst, dass mindestens zwei nach Anspruch 1 geprüfte Langhölzer, die ein für eine Verwendung vorgeschriebenes Qualitätskriterium erfüllen, miteinander unter Bildung eines Balkens mit stehender Klebefuge verklebt werden.

Hierbei ist wesentlich, dass die Langhölzer vor der Bildung zu einem Balken einer Qualitätssicherungsprüfung gemäß Anspruch 1 unterzogen wurden, d.h. über ihre gesamte Länge geprüft sind. Hierdurch ist es möglich, Starkholz - bei zumindest gleicher Qualität zu herkömmlich z.B. aus Brettern gebildeten Balken - einzusetzen.

Eine besonders gute Haftung bzw. Verklebung der Langhölzer miteinander zur Bildung eines Balkens ist dann gewährleistet, wenn die miteinander zur Verklebung gelangenden stehenden Seitenflächen der Langhölzer vor dem Verkleben durch Fräsen bearbeitet werden.

Je nach geforderter Belastbarkeit eines Balkens können auch drei oder mehrere Langhölzer nebeneinander angeordnet zu einem Balken verklebt werden.

Zur Bildung eines Binders aus den unter Anwendung des erfindungsgemäßen Verfahrens hergestellten Balken werden vorzugsweise Balken mit jeweils stehender Klebefuge übereinander gelegt und miteinander zu einem Balkenbinder verklebt.

Ein nach dem erfindungsgemäßen Verfahren hergestellter Balken weist mindestens eine stehende Klebefuge auf, und die die Klebefuge bildenden Seitenflächen sind vorzugsweise durch Fräsen bearbeitet.

Ein Balkenbinder ist zweckmäßig aus zwei oder mehreren übereinander angeordneten und miteinander verklebten Balken gebildet.

So ist ein besonderer optischer Vorteil für die Holzarchitektur gegeben, zumal die stehenden Seitenflächen eines Balkens oder eines Balkenbinders eine Fladenstruktur aufweisen und nicht, wie bei einem Brettschichtholz, nur schmale miteinander verklebte Bretter mit den Klebefugen erkennen lassen.

Vorzugsweise ist ein Balken bzw. ein Balkenbinder aus Langhölzern mit einer Höhe von über 100 mm, vorzugsweise bis ca. 300 mm und einer Breite von über 50 mm, vorzugsweise bis 100 mm gebildet.

Um besonders hohe Festigkeitszonen des Splintbereichs eines Stammes im hochbelasteten Bereich eines Trägers einsetzen zu können, ist nach einer hier offenbarten Ausführungsform ein Balken bzw. Balkenbinder aus Langhölzern mit einer Höhe von über 300 mm, vorzugsweise bis 600 mm und einer Breite von über 50 mm gebildet, wobei Langhölzer eingesetzt sind, deren Schmalseitenbereiche aus Splintholz eines Starkholzes geschnitten sind.

Um auch an der Unter- bzw. Oberseite eines Balkens bzw. Balkenbinders eine Fladenstruktur sicherzustellen, ist zweckmäßig ein Balken bzw. Balkenbinder gekennzeichnet durch die Anordnung und Verklebung mindestens eines Langholzes an der Unterseite und/oder Oberseite, das sich über die gesamte Breite des Balkens bzw. Balkenbinders erstreckt und ebenfalls geprüft ist und nach unten bzw. oben eine Fladenstruktur aufweist.

Eine Einrichtung zur Durchführung der Prüfung umfasst eine Zugkräfte auf ein Langholz aufbringende Zugprüfanlage mit Klemmbacken, deren mit dem Langholz in Eingriff gelangende Seiten mit quer zur Belastungsrichtung verlaufenden Rillen mit bogenförmigem, vorzugsweise kreisbogenförmigem Querschnitt versehen sind.

Zur besseren Kraftübertragung weisen benachbarte Rillen ungleiche Tiefen auf, wobei zweckmäßig benachbarte Rillen Querschnitte mit ungleicher Krümmung sowie ungleiche Breiten aufweisen.

Zur Vermeidung einer Beschädigung des zu prüfenden Langholzes durch die Klemmbacken sind diese so ausgestaltet, dass auf jeweils eine Rille mit größerer Tiefe und Breite und mit geringerer Krümmung eine Rille mit geringerer Tiefe und Breite und mit größerer Krümmung folgt, wobei vorteilhaft Rille an Rille angeordnet ist.

Zur Durchführung des erfindungsgemäßen Verfahrens wird eine Holz-Zugprüfeinrichtung zur Prüfung von in Serie erzeugtem Langholz aus Kantholz, insbesondere aus Konstruktionsvollholz vorgeschlagen, um solches Langholz auch für höherbeanspruchte Konstruktionsteile effizient einsetzen zu können. Insbesondere sollen die derzeit aufgrund der Inhomogenität des Holzes erforderlichen Querschnittszuschläge stark herabgesetzt werden können.

Da in den Wäldern Starkholz überwiegt und der Anteil an Starkholz noch im Steigen begriffen ist, dient die vorgeschlagene Holz-Zugprüfeinrichtung für dieses Starkholz, wobei das Langholz aus Kantholz bzw. Konstruktionsvollholz ausgebildet ist, d.h. nicht aus einzelnen miteinander längsverleimten Holzschichten gebildet ist.

Diese Zugprüfeinrichtung weist folgende Merkmale auf:
- ein Prüfbett, insbesondere Maschinenbett, mit einer Länge entsprechend der maximalen Länge des zu prüfenden Holzstückes,
- einen an einem Endbereich des Prüfbettes angeordneten, vorzugsweise fixiert angeordneten, ersten Spannbock,
- einen entlang des Prüfbettes verbringbaren und auf die Länge des zu prüfenden Holzstückes einstellbaren zweiten Spannbock,
- an beiden Spannböcken vorgesehene Klemmbacken, die ein in Prüflage gebrachtes Holzstück von oben und von unten erfassen und klemmen,
- eine Einrichtung zur Kraftaufbringung auf ein eingeklemmtes Holzstück mittels eines Spannbocks,
- eine Querfördereinrichtung zum Fördern eines Holzstückes von einer seitlich des Prüfbettes angeordneten Pufferzone für Holzstücke zum Prüfbett und weiter zu einer an der der Pufferzone gegenüberliegenden und seitlich des Prüfbettes angeordneten, die geprüften Holzstücke aufnehmenden Lagerzone, sowie
- eine Zentriereinrichtung zum Zentrieren eines in Prüflage gebrachten Holzstückes zwischen den Klemmbacken.

Hierdurch ist es möglich, jedes Holzstück als ganzes zu prüfen und je nach dem Ergebnis der Prüfung für einen bestimmten Einsatz vorzusehen, und zwar ohne Bruchrisiko, wie es z.B. bei Starkholz, wie es derzeit eingesetzt wird, stets gegeben ist.

Hierbei ist vorzugsweise die Querfördereinrichtung von mehreren nebeneinander angeordneten Förderketten gebildet.

Gemäß einer anderen hier offenbarten Ausführungsform weist die Zentriereinrichtung zwei Zentrierarme auf, die von einer Ruheposition oberhalb der oberen Klemmbacke in eine seitlich der Klemmbacken angeordnete Zentrierposition bewegbar sind, vorzugsweise schwenkbar sind, wobei vorteilhaft die Zentrierarme synchron gegen das Holzstück bewegbar sind, und zwar jeweils von einer Seite her.

Bei einer besonders einfachen Einrichtung zur Durchführung einer Längendifferenzmessung ist an jedem Spannbock ein Abtaster zum Anlegen an eine Stirnfläche eines Holzstückes vorgesehen.

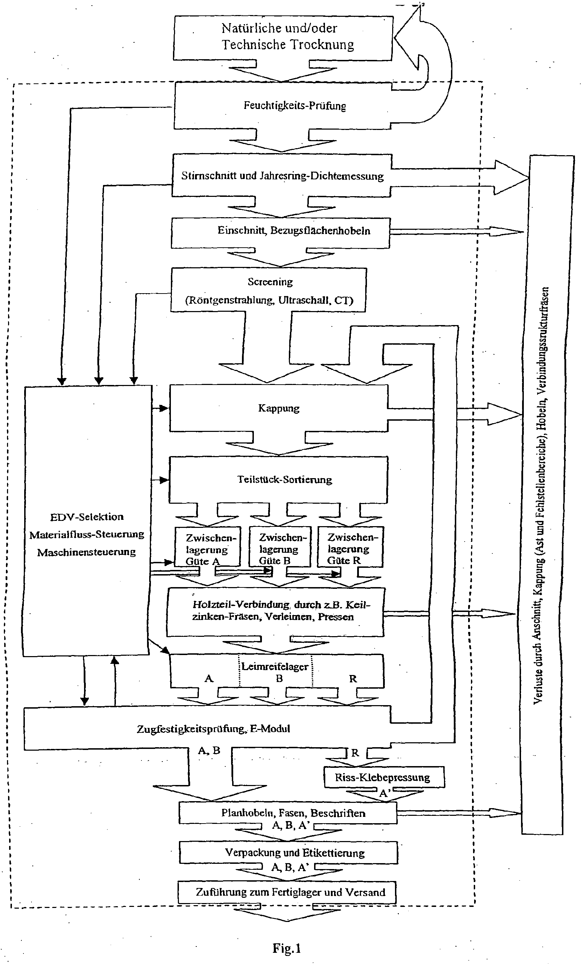

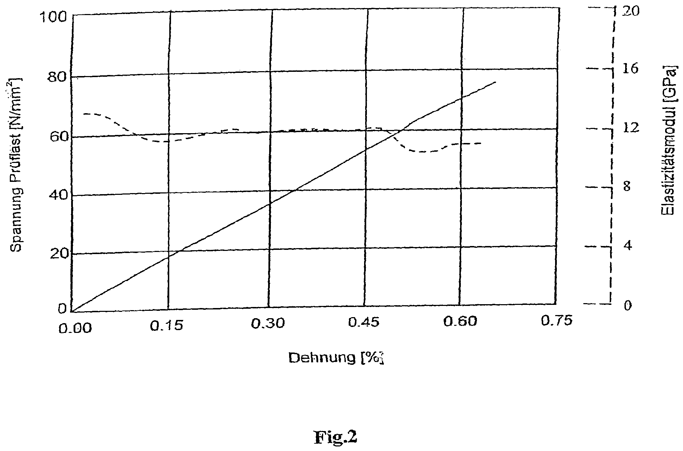

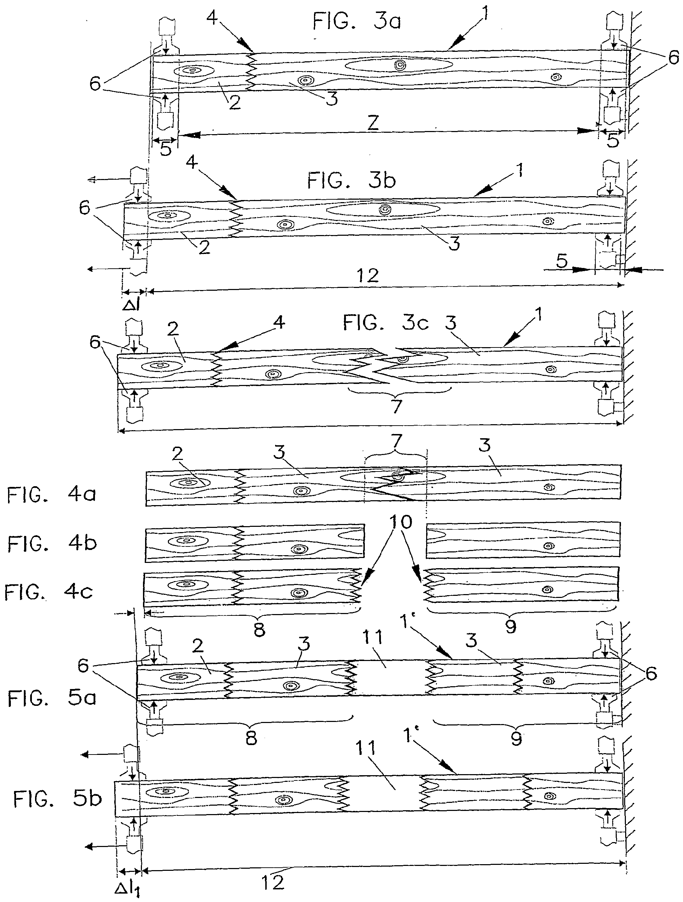

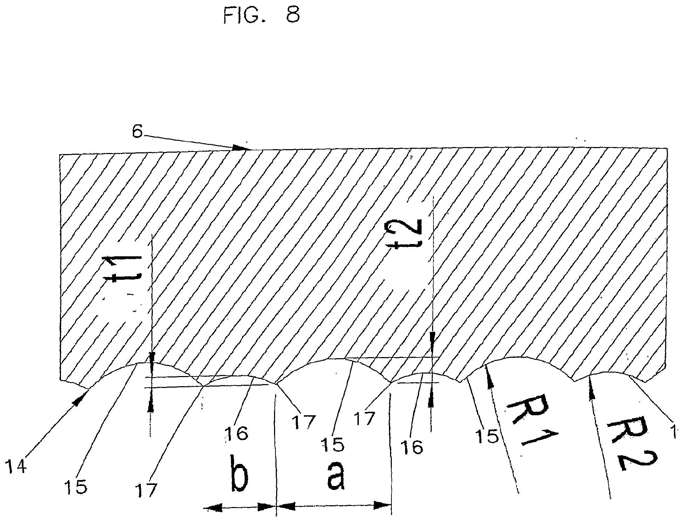

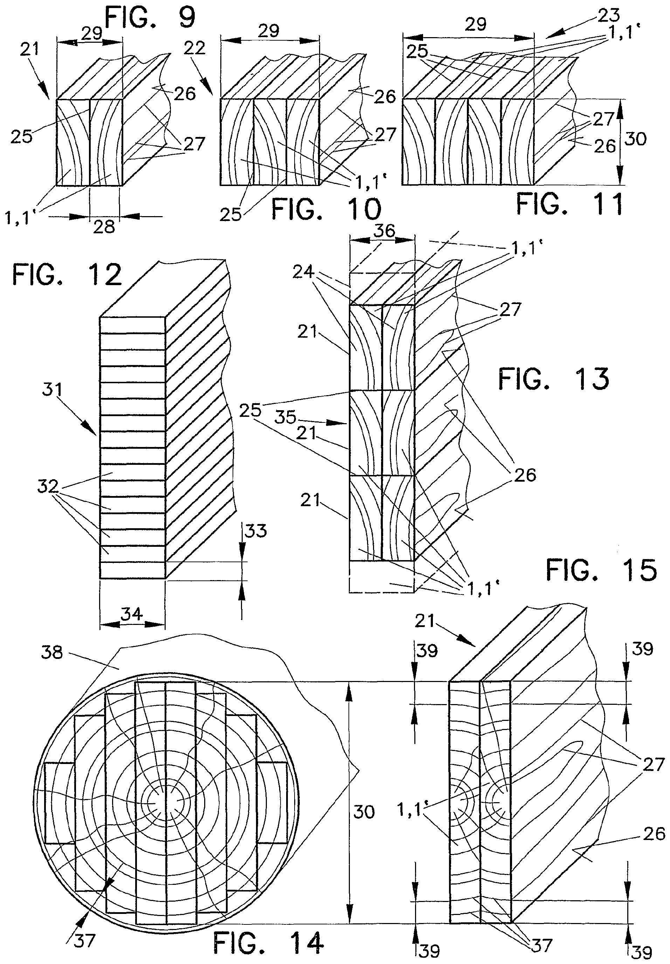

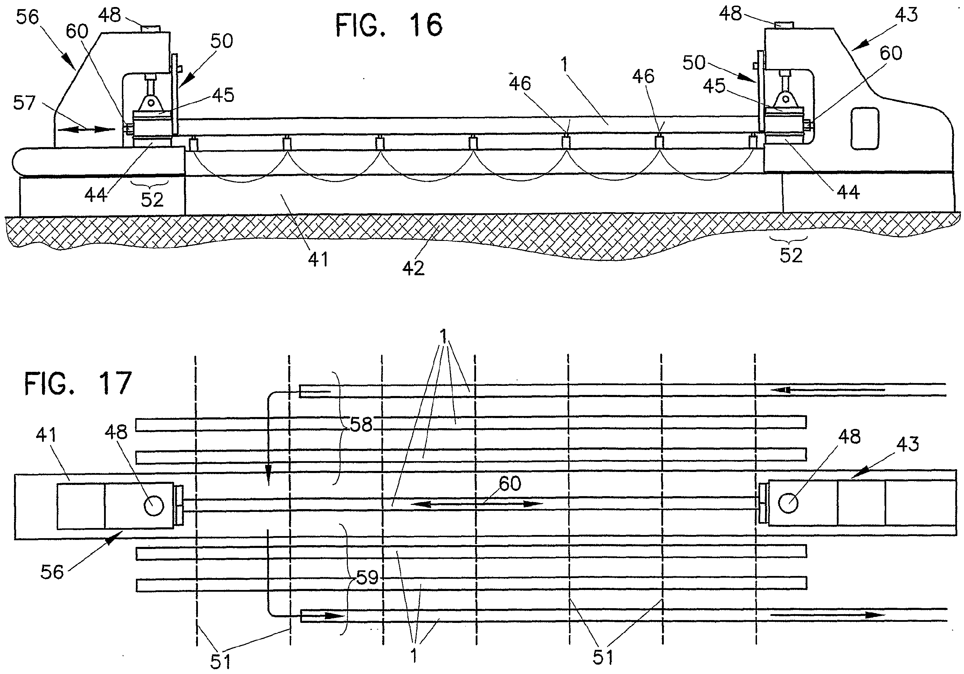

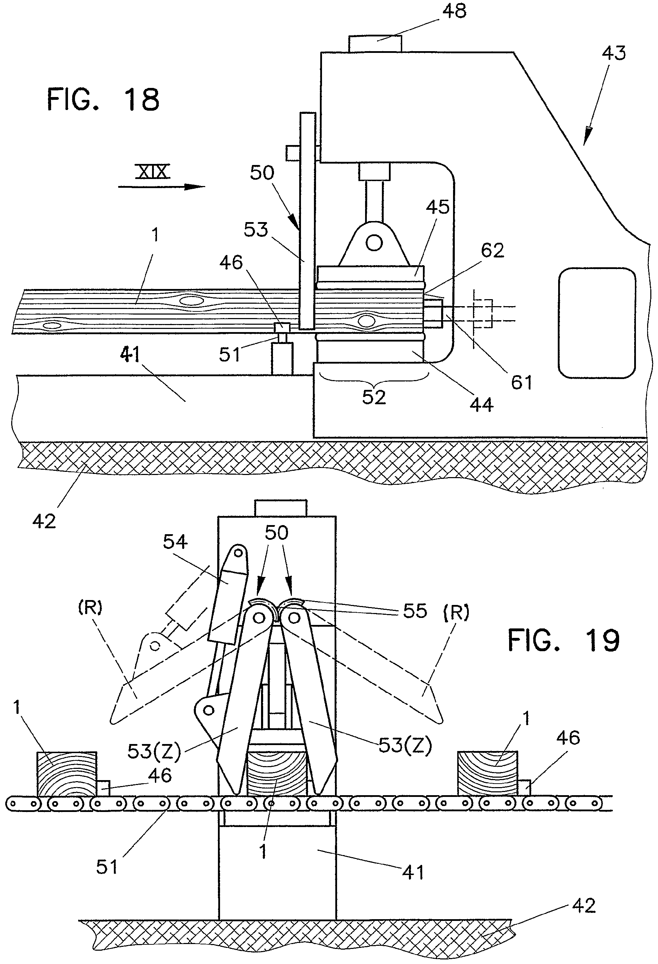

Die Erfindung und nicht als Teil der Erfindung beanspruchte Offenbarungen sind nachstehend anhand der Zeichnung näher erläutert, wobei Fig. 1 die Verfahrensschritte des erfindungsgemäßen Herstellungsverfahrens im Blockdiagramm und Fig. 2 eine typische Zugspannungskurve für ein keilverzinktes Langholz zeigen. Die Fig. 3 bis 7 veranschaulichen die Fertigung und Prüfung von Langhölzern, Fig. 8 eine Klemmbacke im Schnitt. Die Fig. 9 bis 11 zeigen hier offenbarte Balken im Schrägriss. Fig. 12 veranschaulicht einen sogenannten "Leimbinder" aus Brettschichtholz. Die Fig. 13 einen hier offenbarten Balkenbinder jeweils wiederum im Schrägriss. Fig. 14 zeigt ein Starkholz und Fig. 15 wiederum einen Balken im Schrägriß. Fig. 16 betrifft eine Seitenansicht und Fig. 17 eine Draufsicht auf eine Holz-Zugprüfeinrichtung. Die Fig. 18 und 19 zeigen im vergrößerten Maßstab einen Spannbock einmal in Seitenansicht (Fig. 18) und einmal in Richtung des Pfeiles XIX der Fig. 18.

Das durch natürliche (Freilufttrocknung) oder technische Trocknung (z.B. in elektronisch gesteuerten Trockenkammern) auf einen vorbestimmten Wert, wie z.B. auf 15 % ± 3 % Restfeuchtigkeit vorbereitete Holz wird einer Massivholz-Verarbeitungsanlage mittels Stapler oder anderer Fördereinrichtungen zugeführt. Meist dienen Rohholzprodukte in fixer Länge (z.B. 4 m oder auch wesentlich mehr) und bestimmter Querschnittdimensionierung (bestimmter Einschnittsmaße) sowie in möglichst vergleichbarer Qualität als Ausgangsprodukte für eine Standard-Kantholz-Ware.

Jedes Stück dieser Rohware wird zunächst auf den Feuchtigkeitsgehalt hin überprüft. Am genauesten geschieht dies durch die Darrprobe, dabei wird der Gewichtsverlust eines Probestückes bei gezielter Trocknung im Ofen ermittelt. Praktikabler ist die elektrische Widerstandsmessung (Leitfähigkeitsmessung) mittels an zwei oder mehreren definierten Punkten tief in das Holz eindringender Sonden (Rammelektroden). Aber auch kapazitive Methoden (MHz-Bereich) können zur berührungslosen Feuchtigkeitsmessung eingesetzt werden, wobei die Dielektrizitätszahl von der Wasserdichte im Holz abhängig ist. Infrarotmessverfahren, chemisches Messverfahren (z.B. Indikatorpapier), Neutronenstreuverfahren können eingesetzt werden. Auch Mikrowellen-Messverfahren sind zur Feuchtbestimmung möglich. Es können auch Streufeldsensoren, Strahlungsfeldsensoren oder Resonatoren zur Feuchtebestimmung zum Einsatz kommen. Daneben besteht noch die Möglichkeit, über Hygrometer in einem Bohrloch die Feuchtigkeit zu bestimmen.

Die Feuchtigkeitsprüfung ist die erste Selektionsstufe. Zu feuchtes Rohmaterial wird wieder der Trocknung zugeführt.

Ein kurzer Anschnitt an der Stirnfläche dient der sauberen Freilegung der Querschnitt-Struktur zur Vermessung der Jahresring-Dichte. Die Vermessung erfolgt visuell durch Kameras, Laserfokussierung und Bildverarbeitungssoftware oder andere Vermessungseinrichtungen. Diese zweite Stufe der Selektion ermöglicht die automatische EDV-unterstützte Einteilung der Stämme in verschiedene Güteklassen aufgrund der Jahresring-Breiten. Je höher die Dichte der Ringe und je kleiner die Abstände zwischen den Jahresringen, desto höher ist die Festigkeit und damit die Qualität. Äste haben eine festigkeitsreduzierende Eigenschaft, da es zu Störstellen in der Jahresringbildung kommt.

Parallel erfolgt eine Oberflächen-Analyse in Bezug auf Färbungen, Asthäufigkeit, Risse und andere durch Kamera und elektronische Bild-Datenverarbeitung ermittelbare Qualitätskennzeichen. Die so ermittelten qualitativ geeigneten Holzelemente werden anschließend mittels Sägen auf den Rohquerschnitt gebracht, wenn sie bis dahin nicht schon als geeignete vereinzelte Kantholz-Stäbe mit den aufgrund der Trocknungs-Schwindung bedingten Formunebenmäßigkeiten vorliegen. Vorgehobelt wird jedes vereinzelte Rohholz an zumindest zwei Seiten, um klar definierte Bezugsflächen für die weitere Verarbeitung für die Verbindungstechnik zu erhalten.

Zur Ermittlung der inneren Holzqualität wird jedes Holz entweder mit Röngtenstrahlen in einer oder mehreren Richtungen, durch Computer-Tomographie-Technologie mittels Ultraschall einer Echolotung einer Prüfung unterzogen. Die Ergebnisse werden EDV unterstützt mittels Rechner, Computer oder Prozessoren verarbeitet und zur weiteren Verarbeitung des Holzes gespeichert. Hier werden automatisiert, aber auch durch zusätzliche visuelle Kontrolle durch geschultes Personal, alle Fehlstellen für die anschließende Kappung ermittelt und die Daten, wie Kapp-Positionen, Qualitätsstufen, zur weiteren MaschinenSteuerung verwendet.

Geeignete Teilstücke mit Mindestlängen, die sich aus den Einschränkungen durch die Anlage ergeben, werden durch die Kapp-Säge aus den vorselektierten Hölzern herausgeschnitten. Bei ausreichender Eignung wird das Rohmaterial auch ungeteilt der Weiterverarbeitung zugeführt. Die so entstandenen Stücke werden aufgrund der ermittelten Daten der Haupt-Selektion in verschiedene Güteklassen sortiert und getrennt einer oder mehreren Verbindungsanlage(n), wie Keilverzinkungsanlage(n) über Fördereinrichtungen zugeführt.

Die Teilstücke werden in der Regel mittels eines Fräswerkzeuges stirnseitig mit Keilzinken versehen, miteinander verklebt und verpresst und über Fördereinrichtungen in das Klebereifelager gebracht, wo sie wieder nach den der Hauptsortierung entsprechenden Qualitätsstufen getrennt rasten, bis die Klebe-Verbindung die erforderliche Festigkeit aufweist. Alternativ können andere Verbindungstechniken angewendet werden. Daraus resultierende Langhölzer werden in verschiedenen Güteklassen logistisch getrennt gelagert. Jede Qualitätsstufe hat ihre spezifischen Charakteristika, wie Aussehen und Festigkeitsklasse. Die Anzahl der Güteklassen ist beliebig definierbar.

Der einfacheren Erklärung wegen seien drei Qualitätsstufen betrachtet. Qualität A mit hoher Güte, Festigkeit und Eignung für den sichtbaren Bereich (z.B. Normklasse S 13), Qualität B mit Eignung für den nichtsichtbaren Bereich (z.B. Normklasse S 10) und Qualität R mit sichtbaren Rissen aufgrund der Schwindung durch die Trocknung. Je nach Klasse und Holzdimension ergeben sich unterschiedliche maximal zulässige Zugspannungsbelastungen bei der weiteren Zugprüfung.

Die keilverzinkten oder andersartig verbundenen Langhölzer werden über Quer- und Längsförderer vor oder nach dem Planhobeln und Fasern der Endprüfung zugeführt. Dabei wird jedes einzelne Langholz über Klemmeinrichtungen in die Zugprüfungsanlage eingespannt, dann wird die Zuglast bis zur voreingestellten Prüflast (abhängig von Querschnitt und Qualitätsklasse) erhöht und die Längenänderung mittels Messeinrichtung (z.B. nach Laser-Speckle) protokolliert. Daraus wird beispielsweise der Elastizitätsmodul errechnet. Bei Bruch oder Elastizitätsmodulen, die nicht mehr dem Hook'schen Gesetz folgen, wird der Prüfvorgang abgebrochen, die Fehlstelle bzw. Schwachstelle (z.B. schlechte Keilzinkverbindungen) lokalisiert und entfernt und die resultierenden Holzteile in den Produktionszyklus wieder eingeschleust und als Teilstück minderer oder gleicher Qualitätsstufe zugeordnet. Zuvor kann eine Teilung der Stäbe oder Bruchstücke erforderlich sein. Bei unzulässigen Elastizitätsmodulen bzw. zu großer Längenänderung wird das so geprüfte Holz entweder ebenfalls in den Arbeitskreis rückgeführt, wobei eine Teilung und Kappung weiterer vermuteter Schwachstellen erfolgt, oder es wird das Holz für niedrigere Qualitätskriterien geprüft und diesen zugeordnet. Wenn das Holz bricht, so bricht es stets an seiner schwächsten Stelle. Die Bruchstelle wird bei der Rückführung des Ausfall-Holzes in den Produktionsprozess entfernt (gekappt). Dadurch wird prinzipiell ein auf diese Weise zyklisch erzeugtes Langholz mit Verbindungstechnologie, wie Keilzinkverbindungen, von Zyklus zu Zyklus qualitativ besser. Zur Vermeidung von Beschädigungen von Anlage-Einrichtungen und zur Unfallverhütung können Schutzeinrichtungen, wie Schutzkappen, um die Prüfstrecke angeordnet sein, die im Bruchfall (Ausfall) des Prüflings wegsplitternde Teile auffangen. Dennoch lässt sich durch dieses Verfahren mehr Ausbeute aus dem Rohholz, insbesondere Starkholz, erzielen.

Besteht das Produkt die Zugprüfung, wird es mit den Ergebnissen der Prüfung versehen. Dies kann durch Prägung, Bedrucken mit kodierten oder unkodierten Messwerten und anderen Daten, wie Herstellungszeitpunkt, Qualitätsklasse, Firmenbezeichnung u.dgl., direkt auf das Produkt nach dem Planhobeln und Fasern erfolgen oder separat auf Protokollen, die auf die anschließende Verpackung mittels Aufkleber oder direkt aufgebracht werden oder beigelegt werden.

Fig. 2 zeigt ein typisches Spannungs-Dehnungsdiagramm (volle Linie) und seine Ableitung (strichlierte Linie), den Elastizitäts-Modul, für Holz. Während der Zugprüfung werden Zeit, Kraft und Dehnung registriert. Die Auswertesoftware errechnet die Spannung laufend aus dem Kraftsignal und dem bekannten Anfangsquerschnitt.

Es ist vorteilhaft, nach der Feuchtigkeitsprüfung und maschinenunterstützter optischer äußerer und innerer Inspektion die Rohware Stück für Stück und Abschnitt für Abschnitt zu klassifizieren, z.B. wie folgt: A=Top-Qualität; B=Minder-Qualität, R=Rissqualität mit Verwertbarkeit. Unverwertbare Teile werden herausgekappt und aus dem Produktionsprozess genommen. Nach dieser ersten Fertigungsstufe werden die Holzteile nach den oben angegebenen Klassen getrennt. Nun folgt die Holzverbindung. Teil für Teil einer Klasse- bzw. Qualitätsstufe wird durch eine Verbindungstechnik (z.B. Keilzinken) zu einem Strang aneinandergefügt. Nach Erreichen der gewählten Endlänge wird der Strang in die gewünschte Länge des Langholzes - z.B. 8 m - geteilt und die so erhaltenen Langhölzer in den der jeweiligen Qualitätsstufe zugeordneten Klebereife- bzw. Leimreife-Raum befördert. Dieser Vorgang wird für alle Qualitätsstufen wiederholt. Die Produkte der zweiten Fertigungsstufe liegen dann bereits getrennt nach Qualität, jedoch noch ohne Zugversuch vor.

Nach der Reifezeit des Klebers wird produktklassenweise gereiht die Zugprüfung vorgenommen.

Anhand der Fig. 3 bis 7 ist nachfolgend erläutert, wie bei der Prüfung auf Zugfestigkeit vorgegangen wird.

Ein vorgehobeltes Langholz 1, das von zwei Stücken 2, 3, die miteinander mit einer Keilzinkenverbindung 4 verbunden sind, gebildet ist, ist gemäß Fig. 3a an den beiden Enden bzw. Endbereichen 5 mittels paarweise und einander gegenüberliegend angeordneter Klemmbacken 6 fixiert, wobei vorzugsweise ein Klemmbackenpaar 6 an einem Ende 5 des Langholzes 1 ortsfest und das andere Klemmbackenpaar 6, das das andere Ende 5 des Langholzes 1 festspannt, in Achsrichtung des Langholzes zwecks Aufbringung einer Zugkraft bewegbar ist.

Gemäß Fig. 3b ist nach Aufbringen einer Zugkraft auf das Langholz 1 eine erste Längenänderung Δl eingetreten. Eine weitere Steigerung der Zugkraft bis zur Endprüflast führt zu einem Bruch des Langholzes 1, wie dies in Fig. 3 veranschaulicht ist; die Ursache dafür ist eine Schwachstelle 7, die z.B. von einem bei der Vorprüfung nicht erkannten Innenriss etc. gebildet ist. Diese Schwachstelle 7 wird, wie Fig. 4a veranschaulicht, herausgeschnitten (vgl. Fig. 4b) und die beiden restlichen Stücke 8, 9 an den Schnittflächen mit Keilzinken 10 zum abermaligen Aneinanderfügen versehen.

Gemäß Fig. 5a ist zwischen den beiden Stücken 8, 9 ein weiteres Stück 11 eingesetzt, sodass das Langholz 1 trotz der herausgeschnittenen Schwachstelle 7 wiederum die ursprüngliche Länge 12 erreicht. Danach erfolgt abermals eine Zugprüfung bis zur Endprüflast, wie dies in Fig. 5b veranschaulicht ist. Würde die hierbei ermittelte Längenänderung Δl₁ als zulässig eingestuft, hat das nunmehr vorliegende Langholz 1' die Prüfung bestanden.

Es ist zu bemerken, dass die mit voller Last geprüfte Länge sich auf den Abstand Z zwischen den Klemmbackenpaaren 6 beschränkt. Die Endbereiche 5 des Langholzes 1, an dem es von den Klemmbacken gehalten ist, ist nicht unter Volllast geprüft, denn hier nimmt die Zugkraft über die Endbereiche 5 ab.

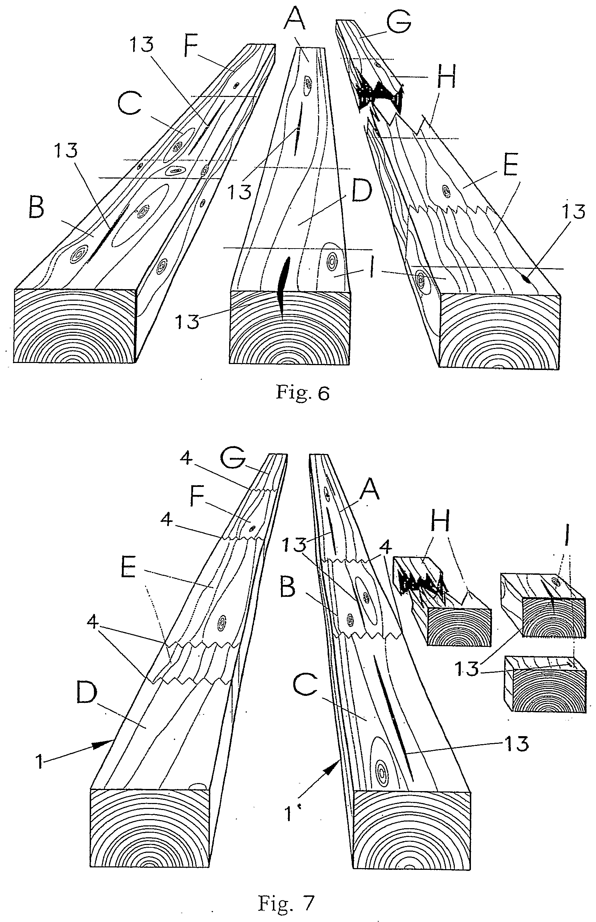

In Fig. 6 sind drei Rohlinge für die Langholzerzeugung im vorgehobelten Zustand nach einer Fehlerprüfung dargestellt. Die Bereiche der Langhölzer A, B, C und I beinhalten Längsrisse 13, und es werden diese Bereiche mittels Keilzinkenverbindungen 4 zu einer eigenen Langholzqualität 1' miteinander verbunden, wie dies rechts in Fig. 7 dargestellt ist, wobei jedoch die Bereiche I in Folge ihrer kurzen Länge Abfall bilden. Die Bereiche D, E, F und G sind ohne erkennbaren Fehler bzw. ohne erkennbare Schwachstelle und werden zur Topqualität mittels Keilzinkenverbindungen 4 zu einem Langholz 1 zusammengefügt. Die beiden Bruchstücke H werden so wie die Bereiche I ausgeschieden. Die Langhölzer 1 und 1' werden sodann der erfindungsgemäßen Zugprüfung unterzogen.

Fig. 8 veranschaulicht eine Klemmbacke 6 im Querschnitt, wie sie zum Aufbringen großer Zugkräfte auf ein Langholz 1, zwecks Bestimmung dessen Längenänderung, am besten geeignet ist. Über die mit dem Langholz 1 in Kontakt gelangende Fläche 14 erstrecken sich Querrillen, also Rillen 15, 16 quer zur Längsrichtung des Langholzes 1 bzw. zur Richtung der Zugkräfte, wobei die Rillen 15, 16 unterschiedliche Tiefen t1 und t2 aufweisen. Vorzugsweise ist neben jeweils einer tieferen Rille 15 eine Rille 16 mit geringerer Tiefe t2 angeordnet, wobei die Übergänge 17 von Rille 15 zu Rille 16 scharfkantig ausgebildet sind.

Die Querschnitte der Rillen 15, 16 sind vorzugsweise teilkreisförmig, wobei die tiefere Rille 15 einen Querschnitt mit etwas größerem Radius R1 aufweist als die Rille 16 mit geringerer Tiefe t2. Das Verhältnis der Breiten a, b der Rillen 15 und 16, also Breite b der weniger tiefen Rille 16 zur Breite a der größeren Rille 15, liegt zwischen 0,3 und 0,6. Das Verhältnis der Radien R1 zu R2 liegt vorzugsweise zwischen 0,8 und 1,5.

Die Fig. 9 bis 11 betreffen sogenannte Balken 21, 22, 23, die aus gemäß Anspruch 1 geprüftem Langholz 1, 1' gebildet sind. Fig. 9 zeigt einen sogenannten Duobalken 21, Fig. 10 einen Triobalken 22 und Fig. 11 einen Quattrobalken 23.

Das wesentliche an diesen Balken 21 bis 23 ist die stehende Klebefuge 25, wodurch sich Seitenflächen 26 mit einer Fladenstruktur 27 ergeben. Die Breite 28 der miteinander zu verklebenden Langhölzer 1,1' beträgt vorzugsweise 60 oder 70 oder 80 mm, wodurch sich Balkenbreiten 29 für einen Duobalken zwischen 120 und 160 mm ergeben. Die Höhe 30 der Langhölzer 1,1' liegt vorzugsweise zwischen 200 und 300 mm. Besonders hoch belastbare Klebefugen 25 ergeben sich, wenn die miteinander zur Verklebung gelangenden stehenden Seitenflächen der Langhölzer 1, 1' vor dem Verkleben durch Fräsen bearbeitet werden.

Die Balkenbreite 29 erstreckt sich stets über mindestens zwei Breiten 28 der Langhölzer 1, 1'; beim Duobalken über zwei, beim Triobalken über drei und beim Quattrobalken über vier Langhölzer 1, 1'.

In Fig. 12 ist ein herkömmlicher "Leimbinder" 31, der aus Brettern 32 als Brettschichtholz gebildet ist, veranschaulicht. Die Höhe 33 der einzelnen Bretter liegt üblicherweise bei 3 bis 4 cm. Die Breite 34 der Bretter 32 liegt üblicherweise zwischen 120 mm und 200 mm. Ein solcher "Leimbinder" 31 ist aufwendig in der Herstellung, und es wird eine große Menge an Leim bzw. Kleber eingesetzt. Seine Seitenansicht lässt sämtliche Klebefugen und vom Holz nur eine schlichte Struktur erkennen.

Anstelle eines solchen "Leimbinders" kann ein Balkenbinder 35 vorgesehen werden, der gemäß Fig. 13 beispielsweise aus Duobalken 21 mit jeweils stehender Klebefuge 25 gebildet ist, wobei drei solche Duobalken 21 übereinandergelegt und miteinander verklebt sind. Die Breite 36 eines solchen Balkenbinders liegt gleichfalls zwischen 120 und 200 mm, kann jedoch auch darüberliegen, beispielsweise wenn Trio- oder Quattrobalken 22 oder 23 übereinandergelegt und miteinander verklebt werden.

Der Vorteil eines solchen Balkenbinders 35 gegenüber einem aus Brettern 32 gebildeten Leimbinder 31 liegt nicht nur in der Optik - bei Seitenansicht ist eine schöne Fladenstruktur 27 zu erkennen -, sondern auch in der Belastbarkeit, zumal er sich aus lauter einzeln und über die gesamte Länge geprüften Langhölzern 1, 1' zusammensetzt. Ein wesentliches Kriterium ist auch, dass er aus Starkholz gebildet sein kann.

Mit strichlierten Linien ist in Fig. 13 angedeutet, dass an der Unter- und/oder Oberseite des Balkenbinders 35 Langhölzer 1,1' angeklebt werden können, sodass auch die Unter- bzw. die Oberseite jeweils eine Fladenstruktur aufweist und damit optisch einer Unterseite eines aus Brettern 32 gebildeten "Leimbinders" 31 entspricht.

Um die besondere Festigkeit von Splintholz 37 für Balken 21 bis 23 bzw. Balkenbinder 35 vorteilhaft ausnützen zu können, wird Starkholz 38 mit Durchmessern von über 400 mm gemäß dem in Fig. 14 dargestellten Schnittplan zerteilt. Hierdurch lassen sich z.B. Langhölzer 1, 1' in der Höhe 30 von etwa 500 bis 600 mm herstellen, deren Schmalseitenbereiche 39 aus dem Aussenbereich, d.h. dem Bereich des Splintholzes 37 eines Starkholzes 38 geschnitten sind.

Verklebt man beispielsweise zwei solche Langhölzer 1, 1' nach einer gemäß Anspruch 1 durchgeführten Qualitätsprüfung zu einem Balken 21, ergibt sich ein besonders hochbelastbarer Balken 21, zumal die Zug- und Druckzone des Balkens von Splintholz 38 gebildet ist, das wie oben erwähnt, eine besonders hohe Festigkeit, insbesondere eine besonders hohe Zugfestigkeit aufweist. Ein Balken 21 dieser Art, wie er z.B. in Fig. 15 veranschaulicht ist, weist ebenfalls eine seitliche Fladenstruktur 27 auf, u.zw. ohne jede Leimfuge. Ein Balken dieser Dimension ist auch als Trambinder bezeichnet.

Balken 21, wie oben beschrieben, sind besonders preisgünstig herstellbar. Es ergibt sich, wie Fig. 14 erkennen lässt, nur wenig Holzverlust und es sind auch nur wenige Arbeitsschritte zu dessen Herstellung notwendig.

Gemäß dem in den Fig. 16 bis 19 dargestellten Beispiel einer Zugprüfmaschine ist ein als Maschinenbett 41 ausgebildetes Prüfbett auf einem Fundament 42 gelagert. Dieses Maschinenbett 41 trägt an einem Ende einen an ihm fixierten bzw. fixierbaren ersten Spannbock 43, der zwei Klemmbacken 44 und 45 aufweist, von denen eine Klemmbacke 44 am Spannbock 43 in der Höhe einer Auflage 46 für das zu prüfende Langholz 1 fixiert ist. Dieser Klemmbacke 44 gegenüberliegend ist die zweite Klemmbacke 45, die mittels einer Krafteinrichtung, wie eines Druckmittelzylinders 48, gegen die untere Klemmbacke 44 unter Klemmung des Langholzes 1 spannbar ist, angeordnet.

An der zum Maschinenbett 41 gerichteten Stirnseite 49 des Spannbocks 41 ist eine Zentriereinrichtung 50 für das zu prüfende Langholz 1 vorgesehen, das mittels einer die Auflage 46 bildenden Querfördereinrichtung 51, die vorzugsweise von mehreren nebeneinander angeordneten Förderketten bzw. Förderbändern gebildet ist, zum Maschinenbett 41 förderbar ist, wobei der Endbereich 52 des Langholzes 1, wie aus Fig. 18 ersichtlich, zwischen den Klemmbacken 44 und 45 zu liegen kommt.

Mittels der Zentriereinrichtung 50, die von zwei am Oberteil des Spannbocks 43 schwenkbar gelagerten Zentrierarmen 53 gebildet ist, lässt sich die Lage gegenüber dem Spannbock 43 genau zentrisch ausrichten. Die Zentrierarme 53 sind mittels einer Stelleinrichtung, wie eines Druckmittelzylinders 54, aus einer oberhalb der Klemmbacken 44 und 45 angeordneten Ruheposition R, die in Fig. 19 mit strichlierten Linien dargestellt ist, in eine Zentrierposition Z synchron schwenkbar, welche Zentrierposition in Fig. 19 mit vollen Linien veranschaulicht ist. Die Synchronbewegung kann beispielsweise mittels ineinandergreifender Zahnsegmente 55, die mit den Zentrierarmen 53 drehfest verbunden sind, verwirklicht sein.

Ein dem oben beschriebenen Spannbock 43 gleich gestalteter, diesem jedoch entgegengesetzt gerichteter, zweiter Spannbock 56 ist am anderen Endbereich des Maschinenbettes 41 vorgesehen. Dieser zweite Spannbock 56 lässt sich gegenüber dem Maschinenbett 41 verbringen (vgl. den Doppelpfeil 57), sodass es möglich ist, Langhölzer 1 mit unterschiedlichen Längen jeweils mit ihren Endbereichen 52 von den Klemmbacken 44, 45 der beiden Spannböcke 43 und 56 zu erfassen.

Seitlich neben dem Maschinenbett 41 ist eine Pufferzone 58 für die zur Prüfung angelieferten Langhölzer 1 vorgesehen, an der vis a vis Seite eine Lagerzone 59 für die bereits geprüften Langhölzer 1, wobei der An- und Abtransport der Langhölzer zu diesen Zonen 58 und 59 jeweils in Längsrichtung der Langhölzer 1 erfolgt. Nach der Prüfung ist vorzugsweise eine Ausscheideklappenanordnung vorgesehen, wo jene Langhölzer mit festgestellter Schwachstelle ausgeschleust werden, um z.B. gekappt wieder in den Prozess zugeführt zu werden.

Zum Aufbringen einer Zugkraft (veranschaulicht durch den Doppelpfeil 60) auf das Langholz 1 nach dessen Klemmung an den beiden Spannböcken 43 und 56, wird zumindest einer der Spannböcke 43, 56 mittels einer Krafteinrichtung, die hier nicht näher dargestellt ist, vom gegenüberliegenden Spannbock wegbewegt.

Um eine Längendifferenz des mit der Prüflast belasteten Langholzes 1 gegenüber dem unbelasteten Langholz 1 festzustellen, werden an den Spannböcken 43 und 56 angebrachte Abtaster 61 gegen die Stirnflächen 62 des Langholzes vor Aufbringen der Prüfkraft bewegt und mit einer vorbestimmten Kraft an diese Stirnflächen 62 während der Prüfung angepresst. Eine Bewegung der Abtaster 61 als Folge einer Längenänderung des Langholzes 1 wird über geeignete Maßeinrichtungen festgestellt und einer Auswertestation übermittelt.

Das Maschinenbett 41 ist im dargestellten Beispiel als aus Stahlblech gefertigter Kastenträger ausgebildet. Selbstverständlich kann es auch vom Fundament 42 selbst gebildet sein, in welchem Fall für den verbringbaren zweiten Spannbock 56 Führungsschienen, die beim dargestellten Beispiel am Maschinenbett 41 vorgesehen sind, am Fundament 42 angeordnet sind.

## Patentansprüche

1. Verfahren zur Qualitätssicherung von in Serie erzeugtem, vorzugsweise keilverzinktem, Langholz (1, 1') aus Kantholz, vorzugsweise aus Konstruktionsvollholz, mit vorbestimmter Mindestlänge (12), **dadurch gekennzeichnet, dass** jedes in der Länge (12) fertig hergestellte Langholz (1, 1') jeweils mit an seinen Enden (5) angreifenden und bis zu einem Grenzwert unterhalb der Bruchlast eines fehlerfreien Langholzes ansteigenden Zugkräften belastet wird.

2. Verfahren nach Anspruch 1, **dadurch gekennzeichnet, dass** eine bei ansteigender Belastung mit Zugkräften ansteigende Längenänderung (Δl₁) in mindestens einem Abschnitt oder über die gesamte Länge des Langholzes (1, 1') bestimmt und als Qualitätskriterium für die Verwendung des Konstruktionsvollholzes (1, 1') bzw. dessen Weiterbehandlung herangezogen wird

3. Verfahren nach Anspruch 2, **dadurch gekennzeichnet, dass** zur Bestimmung der Längenänderung (Δl₁) das Laser-Speckle-Verfahren angewendet wird.

4. Verfahren nach Anspruch 2, **dadurch gekennzeichnet, dass** zur Bestimmung der Längenänderung (Δl₁) ein Differenzlängen-Messverfahren mit direkter Messmethode (skalierte Wegmessung) angewendet wird.

5. Verfahren nach Anspruch 2, **dadurch gekennzeichnet, dass** zur Bestimmung der Längenänderung (Δl₁) eine indirekte Messmethode (z.B. Widerstandsänderung von Dehnmessstreifen) angewendet wird.

6. Verfahren nach Anspruch 1, **dadurch gekennzeichnet, dass** bei einem Bruch des Langholzes (1, 1') die beim Bruch ermittelte Zugspannung für eine Güteklassifizierung der Bruchstücke für deren Verwendung oder Weiterbehandlung herangezogen wird.

7. Verfahren nach einem oder mehreren der Ansprüche 2 bis 5, **dadurch gekennzeichnet, dass** bei Feststellen einer unzulässig hohen Längenänderung des Langholzes (1) bei vorgegebener Zugkraft das Langholz (1) aus der Produktion ausgeschleust, die die unzulässige Längenänderung verursachende Schwachstelle (7) des Langholzes (1) bzw. die Schwachstellen des Langholzes herausgeschnitten und die verbleibenden schwachstellenfreien Teile (8, 9) des Langholzes mittels Keilverzinkung (4) zu einem neuen Langholz, gegebenenfalls unter Hinzufügung weiterer Langholzteile (11), zusammengefügt werden, worauf das neu hergestellte Langholz (1') abermals dem Verfahren nach Anspruch 1 unterworfen wird.

8. Verfahren nach einem oder mehreren der Ansprüche 1 bis 7, **dadurch gekennzeichnet, dass** die Zugkräfte auf das Langholz (1) über an den Enden (5) des Langholzes an zwei einander gegenüberliegenden Seiten vorgesehene Klemmbacken (6) auf das Langholz (1) aufgebracht werden.

9. Verfahren nach einem oder mehreren der Ansprüche 1 bis 8, **dadurch gekennzeichnet, dass** nach der Prüfung eine Fertigbearbeitung des Langholzes (1, 1'), wie z.B. durch Hobeln, Schleifen oder Fräsen, durchgeführt wird.

10. Verfahren nach einem oder mehreren der Ansprüche 1 bis 9, **dadurch gekennzeichnet, dass** in Abhängigkeit des Ergebnisses der Längenänderungsbestimmung die Langhölzer (1, 1') mindestens zwei unterschiedlichen Qualitätsklassen zugeordnet werden.

11. Verfahren nach einem oder mehreren der Ansprüche 1 bis 10, **dadurch gekennzeichnet, dass** das Rohmaterial nach einer Oberflächenanalyse ungeteilt und/oder in Teilstücken geteilt in Güteklassen sortiert und in jeder Güteklasse für sich als Langholz, gegebenenfalls nach Keilverzinken, einem Verfahren zur Qualitätsricherung nach Anspruch 1 unterzogen wird.

12. Verfahren nach einem oder mehreren der Ansprüche 1 bis 11, **dadurch gekennzeichnet, dass** mindestens zwei Langhölzer (1, 1'), die ein für eine Verwendung vorgeschriebenes Qualitätskriterium erfüllen, miteinander unter Bildung eines Balkens (21, 22, 23) mit stehender Klebefuge (25) verklebt werden.

13. Verfahren nach Anspruch 12, **dadurch gekennzeichnet, dass** die miteinander zur Verklebung gelangenden stehenden Seitenflächen der Langhölzer (1, 1') vor dem Verkleben durch Fräsen bearbeitet werden.

14. Verfahren nach Anspruch 12 oder 13, **dadurch gekennzeichnet, dass** drei oder mehr Langhölzer (1, 1') nebeneinander angeordnet und zu einem Balken (22, 23) verklebt werden.

15. Verfahren nach Anspruch 12, 13 oder 14, **dadurch gekennzeichnet, dass** Balken (21, 22, 23) mit jeweils stehender Klebefuge (25) übereinander gelegt und miteinander zu einem Balkenbinder (35) verklebt werden.

## Claims

1. A method for quality assurance of preferably finger-jointed long timber (1, 1'), produced in series and having a predetermined minimum length (12), made of square timber, preferably of structural solid wood, **characterized in that** each long timber (1, 1') finished within the length (12) is loaded with tensile forces engaging the respective ends (5) thereof and increasing to a threshold value below the breaking load of a faultless long timber.

2. A method according to claim 1, **characterized in that** a change in length (Δl₁) increasing with an increasing load with tensile forces is determined in at least one section or across the entire length of the long timber (1, 1') and is used as a quality criterion for the use, or the further processing, respectively, of the structural solid wood (1, 1').

3. A method according to claim 2, **characterized in that** the laser speckle method is used for determining the change in length (Δl₁).

4. A method according to claim 2, **characterized in that** a method of differential length measurement is used with a direct measuring method (scaled path measurement) for determining the change in length (Δl₁).

5. A method according to claim 2, **characterized in that** an indirect measuring method (e.g. resistance change in strain gauges) is used for determining the change in length (Δl₁).

6. A method according to claim 1, **characterized in that**, if the long timber (1, 1') breaks, the tensile stress determined during the breakage is used for classifying the quality of the broken pieces for usage or further processing thereof.

7. A method according to one or several of claims 2 to 5, **characterized in that**, in case an inadmissibly high change in the length of the long timber (1) is determined at a predetermined tensile force, the long timber (1) is eliminated from the production, the weak point (7) of the long timber (1) causing the inadmissible change in length or the weak points of the long timber, respectively, is/are cut out and the remaining parts (8, 9) of the long timber that are free from weak points are assembled by finger jointing (4) into a new long timber, optionally with further long timber parts (11) being added, whereupon the newly produced long timber (1') is again subjected to the method according to claim 1.

8. A method according to one or several of claims 1 to 7, **characterized in that** the tensile forces on the long timber (1) are applied to the long timber (1) via clamping jaws (6) provided at the ends (5) of the long timber on two opposing sides.

9. A method according to one or several of claims 1 to 8, **characterized in that** finish-machining of the long timber (1, 1') such as, for example, by planing, grinding or milling is carried out after the test.

10. A method according to one or several of claims 1 to 9, **characterized in that** the long timbers (1, 1') are allocated to at least two different quality classes depending on the result of the length change determination.

11. A method according to one or several of claims 1 to 10, **characterized in that**, after a surface analysis, the raw material, being undivided and/or divided into sections, is sorted into quality classes and is subjected in each quality class as an individual long timber to a method for quality assurance according to claim 1, optionally after finger jointing.

12. A method according to one or several of claims 1 to 11, **characterized in that** at least two long timbers (1, 1'), which meet a quality criterion required for an application, are glued together, whereby a beam (21, 22, 23) having an upright glued joint (25) is formed.

13. A method according to claim 12, **characterized in that** the vertical side faces of the long timbers (1, 1'), which side faces are glued together, are processed by milling prior to gluing.

14. A method according to claim 12 or 13, **characterized in that** three or more long timbers (1, 1') are arranged side by side and glued together to form a beam (22, 23).

15. A method according to claim 12, 13 or 14, **characterized in that** beams (21, 22, 23) which in each case have an upright glued joint (25) are placed on top of each other and glued together to form a beam binder (35).

## Revendications

1. Procédé de contrôle de la qualité d'une grume (1, 1') produite en série, de préférence aboutée en biais, provenant de bois équarri, de préférence de bois massif de construction, présentant une longueur minimale (12) prédéfinie, **caractérisé en ce que** chaque grume (1, 1') entièrement fabriquée dans la longueur (12) est sollicitée dans chaque cas par des forces de traction s'exerçant à ses extrémités (5) et augmentant jusqu'à une valeur limite inférieure à la charge de rupture d'une grume exempte de défauts.

2. Procédé selon la revendication 1, **caractérisé en ce qu'**une modification de longueur (Δl₁) croissant à mesure que la sollicitation par des forces de traction augmente est déterminée dans au moins une section ou sur toute la longueur de la grume (1, 1') et est utilisée comme critère de qualité pour l'utilisation du bois massif de construction (1, 1') ou le traitement supplémentaire de celui-ci.

3. Procédé selon la revendication 2, **caractérisé en ce que**, pour déterminer la modification de longueur (Δl₁), on utilise le procédé de tavelure laser.

4. Procédé selon la revendication 2, **caractérisé en ce que**, pour déterminer la modification de longueur (Δl₁), on utilise un procédé de mesure de différences de longueur à méthode de mesure directe (mesure de déplacement mise à l'échelle).

5. Procédé selon la revendication 2, **caractérisé en ce que**, pour déterminer la modification de longueur (Δl₁), on utilise une méthode de mesure indirecte (par exemple modification de la résistance de jauges de contrainte).

6. Procédé selon la revendication 1, **caractérisé en ce que**, lors d'une rupture de la grume (1, 1'), la contrainte de traction déterminée lors de la rupture est utilisée pour classer la qualité des morceaux, en vue de leur utilisation ou de leur traitement ultérieur.

7. Procédé selon l'une ou plusieurs des revendications 2 à 5, **caractérisé en ce que**, lorsqu'une modification excessivement élevée de la grume (1) est constatée, dans le cas d'une force de traction prédéfinie, la grume (1) est évacuée de la production, le point faible (7) de la grume (1), lequel est à l'origine de la modification excessive de la longueur, ou les points faibles de la grume sont retirés par découpage et les parties (8, 9) restantes de la grume, exemptes de points faibles, sont réunies par aboutement en biais (4) pour obtenir une nouvelle grume, le cas échéant en ajoutant d'autres parties (11) de grume, la nouvelle grume (1') obtenue étant une nouvelle fois soumise au procédé selon la revendication 1.

8. Procédé selon l'une ou plusieurs des revendications 1 à 7, **caractérisé en ce que** les forces de traction sur la grume (1) sont appliquées sur la grume (1) par l'intermédiaire de mâchoires de serrage (6) prévues aux extrémités (5) de la grume au niveau de deux faces opposées l'une à l'autre.

9. Procédé selon l'une ou plusieurs des revendications 1 à 8, **caractérisé en ce que**, après le contrôle, un finissage de la grume (1, 1'), comme par exemple par rabotage, polissage ou fraisage, est réalisé.

10. Procédé selon l'une ou plusieurs des revendications 1 à 9, **caractérisé en ce que** les grumes (1, 1') sont associées à au moins deux classes de qualité différentes en fonction du résultat de la détermination de la modification de longueur.

11. Procédé selon l'une ou plusieurs des revendications 1 à 10, **caractérisé en ce que** la matière première, après une analyse de la surface, est triée en classes de qualité en étant non séparée et/ou en étant séparée en morceaux et est soumise individuellement dans chaque classe de qualité en tant que grume, le cas échéant après aboutement en biais, à un procédé de contrôle de la qualité selon la revendication 1.

12. Procédé selon l'une ou plusieurs des revendications 1 à 11, **caractérisé en ce qu'**au moins deux grumes (1, 1'), qui satisfont un critère de qualité prescrit pour une utilisation, sont collées ensemble au moyen d'un joint de colle (25) vertical, ce qui entraîne la formation d'une poutre (21, 22, 23).

13. Procédé selon la revendication 12, **caractérisé en ce que** les surfaces latérales verticales collées ensemble des grumes (1, 1') sont usinées par fraisage avant le collage.

14. Procédé selon la revendication 12 ou 13, **caractérisé en ce que** trois grumes (1, 1') ou plus sont agencées les unes à côté des autres et collées pour former une poutre (22, 23).

15. Procédé selon la revendication 12, 13 ou 14, **caractérisé en ce que** les poutres (21, 22, 23) présentant chacune un joint de collage (25) vertical sont posées les unes au-dessus des autres et collées ensemble pour former une poutre composite (35).
